(19) Europäisches Patentamt
European Patent Office
Office européen des brevets

(11) Numéro de publication : **0 454 557 A1**

(12) **DEMANDE DE BREVET EUROPEEN**

(21) Numéro de dépôt : **91401059.0**

(22) Date de dépôt : **22.04.91**

(51) Int. Cl.[5] : **B01J 29/06**, B01J 29/14, C07C 17/154

(30) Priorité : **27.04.90 FR 9005454**

(43) Date de publication de la demande : **30.10.91 Bulletin 91/44**

(84) Etats contractants désignés : **AT BE CH DE DK ES FR GB GR IT LI LU NL SE**

(71) Demandeur : **GAZ DE FRANCE
23, rue Philibert Delorme
F-75017 Paris (FR)**

(72) Inventeur : **Dufaux, Michel
13, Allée des Pervenches
F-69650 Saint Germain au Mont D'Or (FR)**
Inventeur : **Naccache, Claude
20, Chemin de Villeneuve
F-69130 Ecully (FR)**
Inventeur : **Ben Taarit, Yaounès
106, Chemin l'Aigas
F-69160 Tassin la Demi-Lune (FR)**
Inventeur : **Saint Just, Jacques
40 Bis, Avenue du Président Wilson
F-78230 Le Pecq (FR)**

(74) Mandataire : **Durand, Yves Armand Louis et al
CABINET WEINSTEIN 20, Avenue de Friedland
F-75008 Paris (FR)**

(54) **Catalyseurs d'oxyhydrochloruration de méthane.**

(57) La présente invention a pour objet de nouveaux catalyseurs d'oxyhydrochloruration de méthane en chlorure de méthyle.

Les catalyseurs de la présente invention comprennent un composé chimique de grande surface spécifique de type zéolithe qui est partiellement échangée au moins avec des cations métalliques divalents de préférence des cations $Cu^{++}$.

Les catalyseurs de l'invention trouvent notamment application dans la formation de produits monochlorés utilisables en tant que tels ou comme intermédiaires chimiques.

EP 0 454 557 A1

Jouve, 18, rue Saint-Denis, 75001 PARIS

La présente invention a pour objet de nouveaux catalyseurs d'oxyhydrochloruration de méthane en chlorure de méthyle.

L'oxyhydrochloruration est une réaction chimique bien connue qui consiste à faire réagir des alcanes légers avec du chlorure d'hydrogène et de l'oxygène en présence d'un catalyseur pour l'obtention de produits chlorés. Ainsi, l'oxyhydrochloruration du méthane pour la production de chlorure de méthyle s'effectue selon le schéma réactionnel suivant :

$$CH_4 + HCl + 1/2\ O_2 \xrightarrow{\text{catalyseur}} CH_3Cl + H_2O.$$

Le brevet américain n° 4 123 389 révèle notamment l'oxyhydrochloruration du méthane en utilisant un catalyseur constitué d'un support d'oxyde de silicium, d'oxyde de titane ou d'alumine $\alpha$ de surface spécifique élevée sur lequel sont déposés des chlorures de cuivre, essentiellement des chlorures cuivreux, des chlorures de métal alcalin (KCl) et des chlorures de terre rare ($LaCl_3$).

Cependant, des études ont montré que si ce type de catalyseur présente d'assez bonnes activité et sélectivité, il est instable et se décompose facilement lors d'une utilisation prolongée. De plus, la préparation de ce catalyseur exige des précautions draconiennes quant à la proscription de l'eau et nécessite un protocole expérimental strict et difficile à respecter.

Aussi, un objet de la présente invention est de fournir de nouveaux catalyseurs d'oxyhydrochloruration qui présentent de bonnes activité et sélectivité, qui sont chimiquement stables lors d'une utilisation prolongée et qui de plus ne nécessitent pas de précautions draconiennes pour leurs préparation et utilisation.

Les catalyseurs d'oxyhydrochloruration de méthane en chlorure de méthyle de la présente invention comprennent un composé chimique de grande surface spécifique de type zéolithe qui est partiellement échangé au moins avec des cations métalliques divalents, de préférence des cations $Cu^{++}$.

Selon d'autres caractéristiques des catalyseurs de la présente invention, la zéolithe est de préférence une zéolithe de type Y qui à l'état initial répond à la formule générale $Na_{56}\ (AlO_2)_{56}\ (SiO_2)_{136}$ ;

La zéolithe échangée comprend entre environ 0,5 et 10 % en poids de cations $Cu^{++}$, de préférence entre environ 0,5 et 7,6 % en poids et de façon plus préférentielle 4,6 % en poids de cations $Cu^{++}$ ;

La zéolithe est de plus échangée avec des cations $Mn^{++}$ ; et

la zéolithe échangée comprend environ 2 % en poids de cations $Mn^{++}$.

Un autre objet de l'invention est un procédé d'oxyhydrochloruration de méthane en chlorure de méthyle qui consiste à utiliser un catalyseur spécifique.

D'autre buts, détails et avantages de la présente invention apparaîtront plus clairement à la lecture de la description détaillée qui va suivre faite en référence aux exemples non limitatifs et aux figures 1 et 2 dans lesquelles :

La figure 1 représente graphiquement l'activité en % d'un catalyseur de la présente invention et d'un catalyseur de l'art antérieur en fonction du temps.

La figure 2 représente le rendement en % d'un catalyseur de la présente invention et d'un catalyseur de l'art antérieur en fonction du temps.

Les zéolithes sont des alumino-silicates de formule générale $Na_{56}(AlO_2)_{56}(SiO_2)_{136}$. Les atomes de sodium et d'aluminium sont au nombre identique de façon à assurer la neutralité électrique de l'ensemble. Il est possible de faire varier le rapport Si/Al et de remplacer des cations $Na^+$ par d'autres cations tels que par exemple cuivre ($Cu^{2+}$) de façon à obtenir une zéolithe échangée répondant à la formule $Na_{56-x}Cu_{x/2}(AlO_2)_{56}\ (SiO_2)_{136}$ dans laquelle x représente le nombre d'atomes échangés dans la maille élémentaire.

On a échangé plusieurs types de zéolithes selon le processus ci-dessous décrit et on a testé les catalyseurs ainsi obtenus pour établir une comparaison de leurs performances. Les résultats sont résumés dans les tableaux I et II ci-après.

Préparation des catalyseurs.

Une masse donnée de zéolithe est mise en contact avec une quantité nécessaire calculée d'une solution par exemple 1M de sulfate ou nitrate de cuivre assurant le taux d'échange désiré. Pour des taux d'échanges faibles jusqu'à 15 %, le contact peut se faire en une seule fois. La zéolithe est agitée dans la solution à température ambiante pendant environ 8 heures. On constate la décoloration de la solution et la coloration du solide à la décantation. La séparation peut se faire indifféremment par centrifugation ou filtration. La séparation est suivie de plusieurs lavages à l'eau déionisée. Le lavage final doit être très minutieux, et on s'assure par exemple que les eaux de lavage ne contiennent plus d'ions

$SO_4^{2-}$ ou $NO_3^-$.

Comparaison des performances de différents catalyseurs.

Cinq types de zéolithe ont été préparés selon le processus ci-dessus décrit et ont été testés en tant que catalyseurs. La réaction est effectuée à pression atmosphérique tous les 20°C entre 300 et 440°C et pour comparer les catalyseurs les conditions suivantes ont été adoptées :

Débit total : 8 l/h avec l'hélium comme gaz porteur ;

$P_{02} = P_{HCL} = P_{CH4}$ = 12,66 kPa..

Les résultats sont résumés dans les tableaux I et II ci-après et sont donnés pour deux températures de référence : de 360°C et 400°C

TABLEAU I : réaction à 360°C

| Solides | $CH_3Cl$ m.moles $h^{-1}g^{-1}$ | CO m.moles $h^{-1}g^{-1}$ |
|---|---|---|
| YNa/Cu 4,6 % | 1,7 | 0 |
| LNa/Cu 3 % | 1,0 | 0 |
| ZSM5/Cu 2 % | 1,0 | 0 |
| Mordénite Na/Cu 2% | 0,24 | 0 |
| XNa/Cu 3 % | 0,14 | 0 |

TABLEAU II : réaction à 400°C

| Solides | $CH_3Cl$ m.moles $h^{-1}g^{-1}$ | CO m.molesh$^{-1}$g$^{-1}$ |
|---|---|---|
| YNa/Cu 4,6 % | 4,2 (57 %) | 3,2 (43%) |
| LNa/Cu 3 % | 3,4 (60 %) | 2,3 (40%) |
| ZSM5/Cu 2 % | 2,3 (72 %) | 0,9 (28 %) |
| Mordénite Na/Cu 2% | 1,8 (55 %) | 1,5 (45%) |
| XNa/Cu 3 % | 0,6 (67 %) | 0,3 (33 %) |

Comme cela ressort des tableaux ci-dessus la zéolithe Y est la plus performante, mais est cependant moins sélective que d'autres zéolithes à la température de 400°C.

La production de CO peut être attribuée à une oxydation secondaire d'une partie du chlorure de méthyle produit.

Influence de la teneur en cuivre dans les catalyseurs de l'invention.

On a préparé différents catalyseurs de la présente invention en échangeant une zéolithe NaY avec des cations $Cu^{2+}$ comme décrit précédemment et en faisant varier la teneur en $Cu^{2+}$ dans la zéolithe NaY. Les catalyseurs ainsi obtenus ont été testés afin de déterminer leurs performances dans une réaction d'oxyhydrochloruration du méthane.

Les essais ont été réalisés avec un réacteur d'un diamètre interne de 1,2 cm, avec une hauteur de colonne du catalyseur en poudre de 1,5 cm pour une masse maximale disponible utilisée de catalyseur de 0,680 g.

Chaque réaction a été effectuée à une température de 342°C et selon les conditions suivantes :

Pressions : $CH_4$ : 33,72 kPa
$O_2$ : 16,89 kPa
HCl : 16,89 kPa
He : 33,72 kPa
total : 101,30 kPa

$$CH_4/O_2 = CH_4/HCl = 2$$

Débits : $CH_4$ : 1 l/h
$O_2$ : 0,5 l/h
HCl : 0,5 l/h
He : 1 l/h
total : 3 l/h

Les résultats de ces essais sont présentés dans le tableau III ci-après :

TABLEAU III

| Y Na%Cu | $P_{CH_3Cl}$/g cata | Sélectivité en $CH_3Cl$ % | $P_{CO}$/g cata | Sélectivité en CO, % | $P_{CO_2}$/g cata | Sélectivité en $CO_2$, % | Conv.% $CH_4$/g cata | Conv.% HCl/g cata |
|---|---|---|---|---|---|---|---|---|
| 0,5 | 5,4 | 100 | - | - | - | - | 2,1 | 4,25 |
| 1,0 | 6,8 | 92 | 0,6 | 8 | - | - | 2,9 | 5,35 |
| 2,1 | 7,5 | 87 | 1,1 | 13 | - | - | 3,4 | 5,9 |
| 4,6 | 8,6 | 84 | 1,6 | 16 | - | - | 4,0 | 6,77 |
| 7,6 | 6,6 | 84 | 1,3 | 16 | - | - | 3,1 | 5,2 |

cata = catalyseur
P = pression en kPa
conv. = conversion

Les résultats résumés dans le tableau ci-dessus montrent qu'il existe un compromis entre activité et sélectivité et que ce compromis est aux environs d'une teneur en cuivre dans NaY d'environ 4 %.

Comparaison des catalyseurs de la présente invention et de l'art antérieur.

Le catalyseur de brevet américain n° 4 123 389 a été testé dans les mêmes conditions de réaction d'oxyhydrochloruration que celles décrites ci-dessus pour les catalyseurs de la présente invention. Une comparaison a été effectuée entre le catalyseur de la présente invention ayant une teneur en cuivre de 4,6 % en poids et le catalyseur de l'art antérieur.

Les résultats sont résumés dans le tableau IV ci-après :

TABLEAU IV

| Catalyseur | $P_{CH_3Cl}$/g cata | Sélectivité % | | | $P_{CO}$/g cata | Sélectivité CO, % | Conversion % par gramme de cata | |
|---|---|---|---|---|---|---|---|---|
| | | $CH_3Cl$ | $CH_2Cl_2$ $CHCl_3$ $CHCl_4$ | CO | | | $CH_4$ | HCl |
| NaYCu Cu 4,6 % en poids | 8,6 | 84 | 0 | 16 | 1,6 | 16 | 4,0 | 6,8 |
| Brevet américain 4 123 389 (Exemple I) | 9,3 | 93 | 0 | 7 | 0,7 | 7 | 3,9 | 7,3 |

cata = catalyseur

P = pression en KPa

Comme cela ressort du tableau ci-dessus le catalyseur de l'art antérieur apparaît initialement légèrement supérieur au catalyseur de la présente invention puisqu'à conversion sensiblement égale (de l'ordre de 4 %) il est sensiblement plus sélectif en $CH_3Cl$. Cependant la supériorité initiale du catalyseur de l'art antérieur est au mieux de à 10 %.

Comparaison de la stabilité des catalyseurs de l'invention et de l'art antérieur.

Afin de déterminer et de comparer la stabilité du catalyseur de la présente invention et de celui de l'art antérieur, des essais ont été effectués après 24 heures d'activité sur le catalyseur de la présente invention et sur le catalyseur de l'art antérieur, ce dernier ayant été au préalable régénéré suivant le protocole décrit dans le brevet américain n° 4 123 389.

Les résultats obtenus sont résumés dans le tableau V ci-après et aux figures 1 et 2 annexées dans lesquelles les courbes 1 et 3 se rapportent au catalyseur de la prèsente invention et les courbes 2 et 4 au catalyseur de l'exemple I du brevet américain n° 4 123 389. Le rendement en % est le produit de l'activité et de la sélectivité.

TABLEAU V

| Catalyseur | $P_{CH_3Cl}$/g cata | Sélectivité $CH_3Cl$, % | $P_{CO}$/g cata | Sélectivité CO, % | Conversion % | |
|---|---|---|---|---|---|---|
| | | | | | $CH_4$/g cata | HCl/g cata |
| NaY Cu % poids Cu : 4,6 | 8,6 | 84 | 1,6 | 16 | 4,0 | 6,8 |
| Brevet Américain 4 123 389 | 5,9 | 94 | 0,4 | 6 | 2,9 | 4,96 |

P : pression en kPa
cata : catalyseur

Comme cela ressort des tableaux IV et V et des figures 1 et 2 annexées, après 24 heures de mise en oeuvre la performance du catalyseur de l'art antérieur diminue très nettement au niveau de l'activité qui passe de 3,9 % à 2,9 %, avec cependant une légère augmentation de la sélectivité en $CH_3Cl$ qui passe de 93 à 94 %. En fait, cette augmentation n'est pas significative, car en ce qui concerne les réactions d'oxydation partielle, une diminution de conversion doit entraîner une augmentation de la sélectivité sans que le rendement de la réaction soit grandement modifié. Dans le cas présent, le rendement par rapport au méthane passe de 3,6 % à 2,7 % en 24 heures, soit une chute de 25 %, ce qui indique une très rapide désactivation du catalyseur de l'art antérieur. Ce manque de stabilité du catalyseur de l'art antérieur n'est pas surprenant dans la mesure où, dans les conditions de réaction, le catalyseur est probablement sous forme d'un sel fondu de $CuCl_2$ supporté dont une partie est susceptible d'être entraînée par le débit de gaz, ce qui implique une perte de substance.

Par contre, le catalyseur de la présente invention est stable sur la période de 24 heures.

La comparaison des catalyseurs de l'art antérieur et de la présente invention a été étendue à des conditions d'essais différentes, c'est-à-dire dans les conditions suivantes :

Température 342°C,
P totale 101,30 kPa
$P_{CH4}$ 72,38 kPa
$P_{O2}$ = $P_{HCl}$
1 14,43 kPa
$CH_4/O_2$ = $CH_4/HCl$ = 5 (au lieu de 2),
Débit total = 7 l/h
Masse utilisée de catalyseur 0,680 g.

Les résultats sont résumés dans le tableau VI ci-après:

TABLEAU VI

| Catalyseur | $P_{CH3Cl}$/g cata | Sélectivité $CH_3Cl$, % | $P_{CO}$/g cata | Sélectivité CO, % | Conversion, % $CH_4$/g cata | Conversion, % HCl/g cata |
|---|---|---|---|---|---|---|
| NaYCu % poids Cu : 4,6 | 13,8 | 90 | 1,6 | 10 | 2,8 | 12,7 |
| Catalyseur brevet américain 4 123 389 | 15,5 | 91 | 1,5 | 9 | 3,1 | 14,4 |
| NaYCu % poids Cu : 4,6 après 24 h | 13,8 | 90 | 1,6 | 10 | 2,8 | 12,7 |
| Catalyseur brevet américain 4 123 389 après 24 h | 9,5 | 87 | 1,4 | 13 | 2,0 | 7,5 |

cata = catalyseur
P = pression en kPa

Dans ces conditions plus réductrices, le catalyseur de l'art antérieur conserve initialement de meilleures performances que le catalyseur de la présente invention. Cependant, après 24 heures de mise en oeuvre la sélectivité du catalyseur de l'art antérieur baisse accompagnée d'une baisse d'activité alors que le catalyseur de la présente invention est aussi sélectif et actif et devient donc nettement supérieur au catalyseur de l'art antérieur.

En conclusion, dans les conditions réputées les plus favorables au catalyseur de l'art antérieur, celui-ci est effectivement de 8 % plus productif, en raison d'une meilleure sélectivité, que le catalyseur de la présente invention. En revanche, après seulement 24 heures de mise en oeuvre, le catalyseur de la présente invention qui ne subit pas de désactivation lui devient nettement supérieur en productivité (+ 31 %) et en sélectivité (90 contre 87 %).

De plus, il faut noter qu'à de fortes conversions, le catalyseur de l'art antérieur produit des produits chlorés autres que le chlorure de méthyle alors que le catalyseur de la présente invention reste sélectif en chlorure de méthyle.

Enfin, les catalyseurs de l'invention sont préparés simplement par échange ionique sans précaution spéciale et ne nécessitent pas de mesures draconiennes d'utilisation.

Bien que les catalyseurs de la présente invention soient très performants, comme cela ressort de la description qui précède, on a tenté d'améliorer encore leur performance par modification de leurs compositions chimiques. On a donc dopé la zéolithe YNaCu à 4,6 % en poids de cuivre successivement par des ions $Fe^{3+}$, $Ag^{2+}$ et $Mn^{2+}$ qui ont été introduits tour à tour par échange ionique selon le processus précédemment décrit.

Les catalyseurs ainsi obtenus ont été testés respectivement pour deux températures de 360°C et de 400°C et dans les conditions réactionnelles suivantes :

débit total : 8 l/h avec l'hélium comme gaz porteur

$P_{02} = P_{HCL} = P_{CH4} = 12,66$ kPa.

Les résultats obtenus sont présentés dans les tableaux VII et VIII ci-après.

TABLEAU VII

Réaction à 360°C

| Catalyseur | $CH_3Cl$ m moles $h^{-1}g^{-1}$ cata | CO m moles $h^{-1}g^{-1}$cata |
|---|---|---|
| YNa/Cu 4,6 % | 1,67 | - |
| YNa/Cu 4,6 % + 1 % $F_e$ | 2,00 | 0,55 |
| YNa/Cu 4,6 % + 3 % $F_e$ | 1,30 | 0,40 |
| YNa/Cu 4,6 % + 2 % Ag | 0,9 | - |
| YNa/Cu 4,6% + 2 % Mn | 1,4 | - |

Cata : catalyseur

TABLEAU VIII

Réaction à 400°C

| Catalyseur | $CH_3Cl$ m moles $h^{-1}g^{-1}$ cata | CO m moles $h^{-1}g^{-2}$cata |
|---|---|---|
| YNa/Cu 4,6 % | 4,1 (56 %) | 3,2 |
| YNa/Cu 4,6 % + 1 % $F_e$ | 5,2 (47 %) | 5,8 |
| YNa/Cu 4,6 % + 3 % $F_e$ | 4,5 (57 %) | 3,4 |
| YNa/Cu 4,6 % + 2 % Ag | 2,8 | 1,3 |
| YNa/Cu 4,6 % + 3 % Mn | 4,7 (71 %) | 1,9 |

Cata : catalyseur

Il ressort des tableaux ci-dessus que l'échange des ions $Fe^{3+}$ et $Mn^{2+}$ dans la zéolithe YNa/Cu 4,6 % augmente pratiquement toujours l'activité mais que les ions $Fe^{3+}$ (1 à 3 %) entraînent une chute de la sélectivité, probablement due à leur action favorable pour la combustion du chlorure de méthyle tandis que les ions $Mn^{2+}$ engendrent toujours une augmentation de la sélectivité. Quant aux ions $Ag^{2+}$ ils diminuent l'activité mais sans modifier la sélectivité.

On peut donc dire que parmi les cations actifs, les cations $Mn^{2+}$ sont les meilleurs car sans entraîner une diminution de la conversion ni de la stabilité du catalyseur, ils améliorent la sélectivité.

Des essais complémentaires ont été effectués afin d'établir une comparaison entre le catalyseur de base de la présente invention, le catalyseur dopé au manganèse et le catalyseur de l'art antérieur. Les conditions de réaction sont telles que définies pour les essais dont les résultats sont résumés dans le tableau III.

Les résultats sont présentés dans le tableau IX ci-dessous.

TABLEAU IX

Influence du manganèse sur les propriétés catalytiques de la zéolithe Y à 4,6 % de cuivre.

| Catalyseur | Sélectivité, en $CH_3Cl$, % | Conversion, % par gramme de catalyseur | Rendement,% par gramme de catalyseur |
|---|---|---|---|
| Na Cu Mn Y 4,6 % Cu 2,0 % Mn | 98 | 3,3 | 3,3 |
| Na Cu Y 4,6 % Cu | 84 | 4,0 | 3,3 |
| Catalyseur Brevet 4 123 389 (après 24 h) | 94 | 2,9 | 2,7 |

Les résultats du tableau ci-dessus confirment que le dopage du catalyseur de la présente invention au manganèse améliore la sélectivité de celui-ci bien que les rendements par gramme de catalyseurs dopés et non dopés soient identiques. On peut attribuer l'influence bénéfique du manganèse à son action sinon inhibitrice, tout au moins peu catalytique vis-à-vis de la réaction d'oxydation totale du chlorure de méthyle. Quant à la légère baisse d'activité induite par l'addition de manganèse, elle est comparable avec celle qui se produit lorsque le taux global d'échange augmente (voir par exemple NaCuY à 7,6 % en $Cu^{2+}$ dans le tableau III).

En ce qui concerne les avantages du catalyseur de la présente invention modifié par le manganèse, on constate qu'il présente non seulement les avantages du catalyseur de base de la présente invention mais que de plus sa sélectivité en régime permanent est supérieure à celle du catalyseur de base de la présente invention et à celle du catalyseur de l'art antérieur pour une conversion voisine.

## Revendications

1. Catalyseur d'oxyhydrochloruration de méthane, en chlorure de méthyle, caractérisé en ce qu'il comprend un composé chimique de grande surface spécifique de type zéolithe qui est partiellement échangée au moins avec des cations métalliques divalents, de préférence des cations $Cu^{++}$.

2. Catalyseur d'oxyhydrochloruration selon la revendication 1, caractérisé en ce que la zéolithe est de préférence une zéolithe de type Y de formule générale à l'état initial: $Na_{56}$ $(AlO_2)_{56}$ $(SiO_2)_{136}$.

3. Catalyseur d'oxyhydrochloruration selon les revendications 1 et 2, caractérisé en ce que la zéolithe échangée comprend entre environ 0,5 et 10 % en poids de cations $Cu^{++}$, de préférence entre environ 0,5 et 7,6 % en poids de cations $Cu^{++}$, et de façon plus préférentielle 4,6 % en poids de cations $Cu^{++}$.

**4.** Catalyseur d'oxyhydrochloruration selon l'une des revendications précédentes, caractérisé en ce que la zéolithe est de plus échangée avec des cations métalliques divalents $Mn^{++}$.

**5.** Catalyseur d'oxyhydrochloruration selon la revendication 4, caractérisé en ce que la zéolithe échangée comprend environ 2 % en poids de cations $Mn^{++}$.

**6.** Procédé d'oxyhydrochloruration de méthane en chlorure de méthyle, caractérisé en ce qu'il consiste à utiliser un catalyseur comprenant un composé chimique de grande surface de type zéolithe qui est partiellement échangée au moins avec des cations métalliques divalents, de préférence des cations $Cu^{++}$.

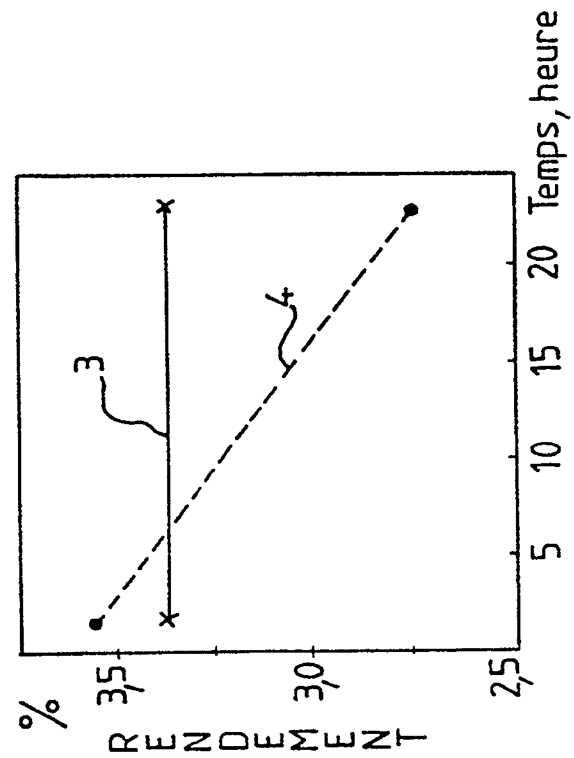
%
RENDEMENT
3,5
3,0
2,5
5
10
15
20
Temps, heure
3
4

Fig:2

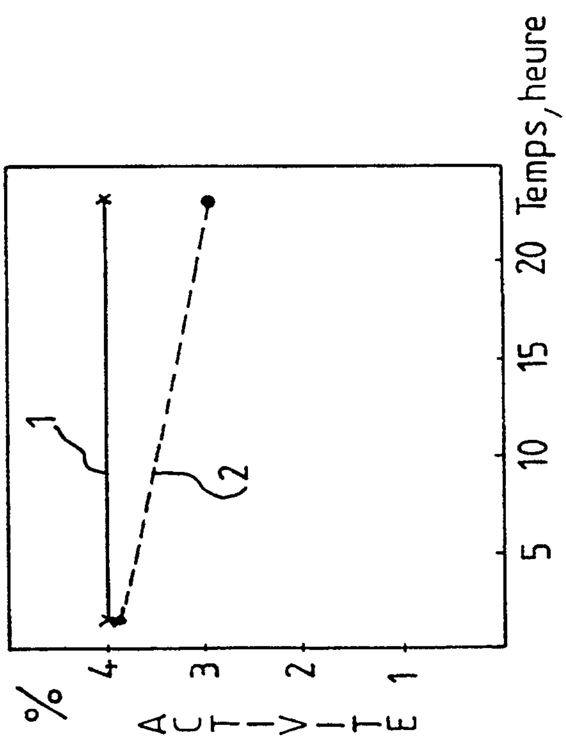
%
ACTIVITE
4
3
2
1
5
10
15
20
Temps, heure
1
2

Fig:1

Office européen des brevets

# RAPPORT DE RECHERCHE EUROPEENNE

Numero de la demande

EP 91 40 1059

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-3 987 118 (KUCK)<br>* Abrégé; colonnes 3-4; revendications 1-18 *<br>--- | 1-6 | B 01 J 29/06<br>B 01 J 29/14<br>C 07 C 17/154 |
| A | GB-A-1 222 249 (ESSO)<br>--- | | |
| A | GB-A-2 095 243 (ICI)<br>--- | | |
| D,A | US-A-4 123 389 (PIETERS et al.)<br>----- | | |
| | | | **DOMAINES TECHNIQUES RECHERCHES (Int. Cl.5)** |
| | | | B 01 J<br>C 07 C |

Le présent rapport a été établi pour toutes les revendications

| Lieu de la recherche | Date d'achèvement de la recherche | Examinateur |
|---|---|---|
| LA HAYE | 29-07-1991 | LO CONTE C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)